# FASCICULE DE BREVET EUROPEEN

(11) **EP 2 055 711 B1**
(45) Date de publication et mention de la délivrance du brevet: **17.08.2011**
(21) Numéro de dépôt: 09152764.8
(22) Date de dépôt: 23.06.2005
(51) Int. Cl.: C07H 19/073, A61K 31/706

(54) **Utilisation de l'acide squalénique pour la formulation d'un principe actif à l'état de nanoparticules**
Einsatz von squalenhaltiger Säure für die Formulierung eines aktiven Prinzips im Nanoteilchenzustand
Use of squalenic acid for the formulation of an active principle in nanoparticle state

(30) Priorité: 30.06.2004 FR 0451365
(43) Date de publication de la demande: 06.05.2009
(62) Demande divisionnaire de: 05857322.1
(73) Titulaire: Centre National de la Recherche Scientifique, 75794 Paris Cedex 16 (FR); Université Paris Sud (Paris 11), 91405 Orsay (FR); Rocco, Flavio, 10042 Nichelino TO (IT)
(72) Inventeur: Rosilio, Véronique, 91140 Villebon-Sur-Yvette (FR); Renoir, Jack-Michel, 94240 L'Hay-Les-Roses (FR); Couvreur, Patrick, 91140 Villebon-Sur-Yvette (FR); Rocco, Flavio, 10042 Nichelino TO (IT); Cattel, Luigi, 10133 Torino (IT); Stella, Barbara, 10134 Torino (IT)
(74) Mandataire: Le Coupanec, Pascale A.M.P.

(56) Documents cités:
- EP-A- 0 693 077
- WO-A-98/32762
- US-B1- 6 384 019
- US-B1- 6 524 595
- WARNER, DONALD T. ET AL: "Nucleic acids. 13. 3'-0- and 2'-0-esters of 1-.beta.-D- arabinofuranosylcytosine as antileukemic and immunosuppressive agents" JOURNAL OF MEDICINAL CHEMISTRY ( 1972 ), 15(8), 790 -2 CODEN: JMCMAR; ISSN: 0022-2623, 1972, XP002473874
- KAWAGUCHI, TAKEO ET AL: "Ester prodrugs of zidovudine" JOURNAL OF PHARMACEUTICAL SCIENCES ( 1990 ), 79(6), 531 3 CODEN: JPMSAE; ISSN: 0022-3549, 1990, XP002473875
- KAWAGUCHI, TAKEO ET AL: "Prodrugs of 2',3'-dideoxyinosine (DDI): improved oral bioavailability via hydrophobic esters" CHEMICAL & PHARMACEUTICAL BULLETIN ( 1992 ), 40(5), 1338, 40 CODEN: CPBTAL; ISSN: 0009-2363, 1992, XP009097748

## Description

La présente invention concerne selon un premier aspect, l'utilisation de l'acide squalénique ou de l'un de ses dérivés hydrocarbonés au moins en . C18, acycliques, non linéaires et insaturés, pour formuler un principe actif de nature polaire avec un poids moléculaire supérieur ou égale à 100 Da, en particulier supérieur à 150 Da, plus particulièrement supérieur à 200 Da, à l'état de naoparticules, ledit principe actif étant un nucléoside ou l'un de ses analogues.

Cette utilisation implique notamment le couplage, en particulier covalent, d'au moins une molécule d'acide squalénique ou de l'un de ses dérivés hydrocarbonés au moins en C18 acycliques, non linéaires et insaturés, à une molécule de principe actif considéré.

En effet, les inventeurs ont montré que des nucléosides antiviraux associés de façon covalente à un dérivé squalénique pouvaient former des nanoparticules. Cet aspect est plus particulièrement illustré par les exemples 2, 3, 4 et 5.

A titre illustratif et non limitatif des nucléosides antiviraux ou analogues structuraux susceptibles d'être formulés selon l'invention, on peut notamment citer la didanosine, la zidovudine et l'acyclovir mais aussi la zalcitabine, le gancyclovir, le _ valacyclavir, la stavudine, la lamivudine, l'abacavir, l'emtricitabine, l'amdoxovir, le dOTC ou même le sidophovir.

La présente invention concerne également selon un autre de ses aspects, l'utilisation de ces dérivés et nanoparticules pour la préparation d'une composition pharmaceutique dotée d'une activité anticancéreuse ou antivirale.

Elle concerne en outre une composition pharmaceutique comprenant, à titre de matière active, au moins un dérivé conforme à la présente invention, notamment sous la forme de nanoparticules.

Conviennent tout particulièrement à l'invention, les dérivés hydrocarbonés acycliques, non linéaires et insaturés à l'image des dérivés terpéniques comme par exemple le squalène et ses dérivés.

Avantageusement, ce dérivé hydrocarboné est un acide carboxylique. Auquel cas, cette liaison covalente est plus particulièrement de nature ester dans le cas des groupements 3' ou 5'-OH, et amide dans le cas du groupement 4-amino.

Bien entendu, les dérivés selon la présente invention peuvent être des dérivés comprenant deux dérivatisations, voire trois dérivatisations, celles-ci pouvant être identiques ou différentes.

Au sens de l'invention, le terme dérivé de squalénoyle entend couvrir les dérivés de substitution du radical squalénoyle dans la mesure où la présence de ce ou ces substituant(s) n'a pas d'incidence significative sur la conformation d'origine du radical. En d'autres thermes, le radical doit conserver son aptitude à se compacter ou encore à provoquer une diminution significative de la tension superficielle ou encore chute rapide de la tension superficielle, lorsqu'il est mis en présence, à partir d'une certaine concentration, avec un solvant polaire.

De manière inattendue, les inventeurs ont ainsi constaté que les dérivés conformes à la présente invention et comprenant, à titre de radical hydrocarboné un radical squalénoyle, s'avéraient particulièrement sensibles à la polarité des solvants, à l'image du squalène. Ils ont ainsi constaté que là mise en présence de ces dérivés avec un solvant polaire à l'image de l'eau par exemple, conduisait à la formation spontanée de particules à l'échelle du nanomètre et donc avantageusement compatibles avec une administration intraveineuse.

Les nanoparticules selon la présente invention s'avèrent accessibles à l'aide de la technologie classique de nanoprécipitation telle que décrite dans Fessi H. et al., Int. J. Pharm., 55 ; 1989, R1-R4.

Plus précisément, les nanoparticules selon l'invention sont obtenues par solubilisation d'un dérivé conforme à la présente invention dans un solvant organique à l'image de l'acétone et/ou de l'éthanol. L'ajout du mélange, ainsi obtenu, dans une phase aqueuse sous agitation conduit instantanément à la formation des nanoparticules attendues en présence ou non de tensioactif(s).

De manière avantageuse, le procédé ne requiert pas la présence obligatoire de tensioactif(s) pour obtenir des nanoparticules conformes à l'invention. Cette propriété est particulièrement appréciable dans la mesure où un grand nombre de tensioactifs ne s'avèrent pas compatibles avec une application in vivo.

Toutefois, il est entendu que l'usage de tensioactifs, généralement avantageusement dénués de toute toxicité, est envisageable dans le cadre de la présente invention. Ce type de tensioactif peut par ailleurs permettre d'accéder à des tailles encore plus réduites lors de la formation des nanoparticules.

A titre illustratif et non limitatif de ce type de tensioactifs susceptibles d'être utilisés dans la présente invention, on peut notamment citer des copolymères de polyoxyéthylène-polyoxypropylène, des dérivés phospholipidiques et des dérivés lipophiles du polyéthylène glycol. Comme dérivé lipophile du polyéthylène glycol, on peut mentionner par exemple le polyéthylène glycol cholestérol.

Comme exemple des copolymères blocs polyocyéthylène-polyoxypropylène, on peut plus particulièrement citer les copolymères triblocs polyoxyéthylène-polyoxypropylène-polyoxyéthylène, encore appelés Poloxamers^{®}, Pluronics^{®} ou Symperonics^{®}, et qui sont commercialisés notamment par la société BASF. Apparentés à ces familles de copolymères, les Poloxwnines^{®}, qui sont constituées de segments hydrophobes (à base de polyoxypropylène), de segments hydrophiles (à base de polyoxyéthylène) et d'une partie centrale dérivant du motif éthylène diamine, peuvent également être employés.

La suspension colloïdale de particules peut être conservée telle quelle, voire évaporée de manière à concentrer les nanoparticules selon l'invention.

D'une manière générale, les nanoparticules ainsi obtenues possèdent une taille moyenne en poids variant de 30 à 500 nm, et en particulier de 50 à 250 nm, notamment de 70 à 200 nm, et voire de 100 à 175 nm mesurée par diffusion de la lumière à l'aide du nanosizer Coulter^{®} N4MD, Coulter Electronics, Hialeah, USA.

Cette aptitude des dérivés selon l'invention à conduire à la formation de nanoparticles, est de toute évidence le résultat d'un comportement spécifique de ces dérivés en milieu aqueux.

Les dérivés conformes à la présente invention peuvent également être administrés par toutes les voies conventionnelles. Toutefois, comme précisé précédemment ces compositions sont particulièrement intéressantes notamment lorsqu'elles sont sous la forme nanoparticulaire pour une administration parentérale.

Un autre aspect, de l'invention concerne donc une composition pharmaceutique comprenant au moins, au titre de matière active, un composé conforme à la présente invention notamment sous la forme de nanoparticules. Les dérivés conformes à la présente invention peuvent y être associés avec au moins un véhicule pharmaceutiquement acceptable.

A titre d'exemples de formulations pharmaceutiques compatibles avec les compositions selon l'invention on peut notamment citer :
- les injections ou perfusions intraveineuses ;
- les solutions salines ou d'eau purifiée ;
- les compositions pour inhalation ;
- les compositions pour voie oculaire ;
- les capsules, dragées et cachets incorporant notamment à titre de véhicules de l'eau, du phosphate de calcium, des sucres, tels que lactose, dactrose ou mannitol, du talc, de l'acide stéarique, de l'amidon, du bicarbonate de sodium et/ou de la gélatine.

Lorsque les composés sont utilisés en dispersion dans une solution aqueuse, ils peuvent être associés à des excipients de type agent séquestrant ou chélatant, antioxydant, agents modifiant le pH et/ou agents tampons.

Les nanoparticules selon l'invention sont bien entendu susceptibles de porter en surface une multitude de fonctions réactives, à l'image des fonctions hydroxyles ou amines par exemple. Il est donc envisageable de fixer à ces fonctions toutes sortes de molécules, notamment par des liaisons covalentes.

A titre illustratif et non limitatif de ce type de molécules susceptibles d'être associées aux nanoparticules, on peut notamment citer les molécules de type marqueur, les composés susceptibles d'assurer une fonction de criblage, ainsi que tous composés aptes à leur conférer des caractéristiques pharmacocinétiques particulières. En ce qui concerne ce dernier aspect, on peut ainsi envisager de fixer en surface de ces nanoparticules des dérivés lipophiles du polyéthylène glycol, comme par exemple le polyéthylène glycol cholestérol ou le polyéthylène glycol-phosphatidiléthanolamine. Un enrobage de surface à base d'un tel composé est en effet avantageux pour conférer une rémanence vasculaire accrue en raison d'une réduction significative de la capture des nanoparticules par les macrophages hépatiques.

Il est en outre possible d'envisager l'association par voie non covalente de composés conformes à la présente invention et/ou des nanoparticules correspondantes avec des molécules annexes telles que définies précédemment. Cette association peut par exemple relever de phénomènes d'adsorption dus notamment à une affinité entre les composés selon l'invention et ces autres molécules.

Outre les composés précités, les compositions pharmaceutiques selon l'invention peuvent contenir des agents de type conservateurs, des agents mouillants, des agents solubilisants, des agents de coloration, et les parfums.

Pour des raisons évidentes, les quantités en dérivés selon l'invention susceptibles d'être mis en oeuvre notamment à des fins anti-cancéreux sont susceptibles de varier significativement selon le mode d'utilisation et la voie retenue pour leur administration.

Par exemple pour un traitement par voie systémique, destiné à un patient de type adulte, on peut envisager d'administrer un dérivé conforme pour la présente invention à une dose d'environ 0,1 à 150 mg/kg de poids corporel et par jour et plus particulièrement de 1 à 40 mg/kg par jour.

En revanche pour une administration topique on peut envisager de formuler au moins un dérivé conforme à la présente invention à raison de 0,1 à 40 % en poids, voire plus, par rapport au poids total de la formulation pharmaceutique considérée.

Il est également possible de co-administrer au moins un dérivé conforme à la présente invention avec au moins une autre matière active, susceptible d'être également bénéfique à l'égard de la pathologie considérée.

A titre représentatif de ces matières actives, susceptibles d'être combinées aux dérivés, conformes à la présente invention, on peut notamment citer d'autres molécules ou macromolécules anticancéreuses ou cytostatiques (par exemple sels de platine, antracyclines, poisons du fuseau mitotique, inhibiteurs de topoisomérases, de kinases ou de métalloprotéases), des agents anti-inflammatoires de type corticoïde (par exemple dexamétasone) ou non corticoïde ou encore des molécules à activité immunoadjuvantes (par exemple anticorps à activité anticancéreuse). L'association avec l'hyperthermie utilisée dans certaines chimiothérapies peut être envisagée. Les dérivés conformes à la présente invention peuvent également être combinés aux thérapies chirurgicales et/ou aux radiations pour le traitement du cancer.

### Exemple 1: Préparation de la 4-(N)-squalénoylgemcitabine (SQuem) (Hors invention)

### a) Synthèse de l'acide squalénique (SOCOOH)

A 11 ml d'eau distillée sont ajoutés 1,16 ml d'acide sulfurique; ensuite, 0,615 g (2,06 mmol) de Na₂Cr₂O₇·2H₂O sont ajoutés doucement afin d'obtenir l'acide chromique. 0,794 g (2,06 mmol) de l'aldéhyde squalénique (SQCHO) (Ceruti M. et al., J. Chem. Soc., Perkin Trans, 1 ; 2002, 1477-1486) sont dissous dans 16 ml d'éther diéthylique sous agitation magnétique et le ballon est ensuite refroidi à 0° C. Ensuite, à la solution de SQCHO est ajouté l'acide chromique goutte à goutte. La réaction est maintenue sous agitation magnétique à 0° C pendant deux heures. Le produit brut est ensuite purifié par lavage de la phase organique avec de l'eau et ensuite par flash-chromatographie sur gel de silice avec éther de pétrole/éther 95:5 comme éluent. Rendement: 35 % (0,286 g, 0,714. mmol).

¹H RMN (CD₃COCH₃ 99.5% 300 MHz) δ : 5.11 (5H, m, CH vinyliques), 2,38 (2H, t, CH₂*CH₂*COOH), 2,26 (2H, t, *CH₂*CH₂COOH), 2,13-1,86 (16H, m, CH₂ allyliques), 1,65-1,59 (15H, m, CH₃ allyliques), 1.26 (3H, s, CH₃ allyliques).

CIMS (isobutane) *m*/*z* 401 (100).

EIMS *m*/*z* 400 (5), 357 (3), 331 (5), 289 (3), 208 (6), 136 (3), 81 (100).

### b) Synthèse de la 4-(N)-squalénoylgemcitabine

Dans un ballon à trois cols muni d'un fluxmètre, 0,209 g (0,522 mmol) de SQCOOH obtenus en a) dissous dans 1 ml tétrahydrofurane (THF) anhydre et ensuite 0,053 g (0,522 mmol) de triéthylamine (TEA) dissous dans 0,5 ml THF anhydre sont ajoutés sous agitation magnétique et avec un flux d'argon. Le ballon est ensuite refroidi à -15°C. Au mélange de réaction, 0,057 g (0,522 mmol) d'éthylchloroformiate dissous dans 2,15 ml THF anhydre sont ajoutés goutte à goutte. Après 20 minutes à -15° C, 0,137 g (0,522 mmol) de gemcitabine dissous dans 2,72 ml de diméthylformamide (DMF) sont ajoutés et la température est augmentée jusqu'à +5°C et finalement à température ambiante. La réaction est suivie par chromatographie sur couche mince (dichlorométhane/acétone 50:50) et elle est gardée sous agitation magnétique pendant plusieurs jours jusqu'à la formation de l'amide. Le produit brut est ensuite purifié par flash-chromatographie sur gel de silice avec un mélange dichlorométhane/acétone 95:5 comme éluent. Rendement: 55 % (0,185 g, 0,287 mol).

¹H RMN (pyridine-d₅ 99.5% 300 MHz) δ: 12,05 (1H, s, NHCO), 8,77 (1H, d, CH-6), 7,74 (1H, d, CH-5), 6,99 (1H, t, CH-1'), 5,30-5,02 (1H, m, CH-3' et 5H, m, CH vinyliques), 4,47-4,31 (3H, m, CH-4' et CH₂-5'), 2,81 (2H, t, NHCO*CH2*), 2,53 (2H, t, NHCOCH₂*CH₂*), 2,18-2,00 (16H, m, CH₂ allyliques), 1,68-1,55 (18H, m, CH₃ allyliques).

CIMS (isobutane) *m*/*z* 646 (100).

EIMS *m*/*z* 645 (10), 577 (8), 523 (7), 509 (18), 494 (10), 454 (15), 429 (24), 372 (100).

### c) Préparation des nanoparticules constituées de 4-(N)-squalénoylgemcitabine

Les particules constituées de SQgem sont obtenues selon la technique de la nanoprécipitation décrite dans Fessi H. et al, Int. J. Pharm., 55 ; 1989, R1-R4. Un échantillon d'une solution à 10 mg/ml de SQgem dans l'éthanol est prélevé et ajouté à de l'acétone selon la concentration désirée et d'une façon telle à obtenir un total de 2 ml de phase organique. Cette solution de SQgem dans le mélange éthanol/acétone est ensuite ajoutée à 4 ml d'eau MilliQ^{®} sous agitation magnétique. Les particules se forment instantanément. Après évaporation sous vide des solvants organiques, une suspension de particules colloïdales stables de SQgem est obtenue. La suspension doit être conservée à +4° C.

### Exemple 5-2: Préparation des nanoparticules constituées de 4-(N)-_ squalénoyleytarabine (SQara-C)

La 4-(*N*)-squalénoylcytarabine est synthétisée à partir de l'acide squalénique par réaction avec là cytarabine, selon la procédure décrite dans l'exemple 1 pour la 4-(*N*)-squalénoylgemcitabine. Les particules constituées de SQara-C sont obtenues selon la technique de la nanoprécipitation, comme décrit pour les particules de SQgem, et leur diamètre hydrodynamique moyen est de 110,4 ± 34,1 nm pour une concentration en SQara-C dans la suspension finale de 1 mg/ml (index de polydispersité : 0,168)

### Exemple 3 : Synthèse des nanoparticules de la 5'-squalenoyl-didanosine; (2S,5R)-((4,8,13,17,21-Pentamethyl-docosa-4,8,12.16,20-pentaenoate de 5-(6-oxo-1,6-dihydro-purin-9-yl)-tétrahydro-furan-2-yl-méthyle

A une solution de 31 mg d'acide (4,8,13,17,21-pentaméthyl-docosa-4,8,12,16,20-pentaenoïque (SqCO2H 0,15 mmol) dans le diméthylformamide anhydre (1,2 mL) sont ajoutés 28 mg de N-hydroxybenzotriazol (0,18 mmol), 36 mg de didanosine (ddI, 0,15. mmol), 70 mg de O-(7-azabenzotriazol-1-yl)-N,N,N',N'-tetramethyluronium hexafluoroborate (0,18 mol) et finalement 62 mg de diisopropyléthylamine (0,5 mmol). Le mélange est agité 84 h à 20 °C sous atmosphère d'azote, puis concentré sous pression réduite (0,05 Torr). Le résidu est repris dans 5 mL d'une solution aqueuse saturée en bicarbonate de sodium, et extrait à l'acétate d'éthyle (3 X 10 mL). La phase organique est lavée par une solution aqueuse de NaCl, séchée sur MgSO4, et concentrée sous vide. Le résidu est chromatographié sur gel de silice (CH2Cl2/MeOH: 92/8) pour fournir 37 mg de 5'-squalenoyldidanosine (Rdt 58%) sous la forme d'un solide amorphe incolore.

IR (cm-1) 3550-2700, 2921, 2856, 1734, 1691, 1590, 1548, 1449, 1380, 1261.

¹H RMN (200 MHz, CDC13) δ: 13.0 (s large, 1H), 8.18 (s, 1H), 8.08 (s, 1H), 6.38 (t, J = 4,2 Hz, 1H), 5,17-5,00 (m, 5H), 4,40-4,20 (m, 3H), 2,60-1,90 (m, 24 H), 1,67 (s, 3H), 1.60 (s large, 15 H).

¹³C RMN (50MHz, CDCl3) δ : 173.27 (CO2), 159.20 (CO), 148.34 (C), 144.3 (CH) 138.60 (CH), 135.23 (C), 135.03 (C), 135.00 (C), 133.09 (C), 131.31 (C), 125.56 (CH), 125.38 (C), 125.54 (CH), 124.53 (CH), 124.40 (2 CH), 85.94 (CH), 79.60 (CH), 65.07 (CH2), 39.86 (CH2), 39.83 (CH2), 39.68 (CH2), 34.67 (CH2), 33.12 (CH2), 33.01 (CH2), 28.39 (CH2), 28.38 (CH2), 29.9 (CH2), 26.83 (CH2), 26.79 (CH2), 26.28 (CH2), 25.77 (CH3), 17.77 (CH3), 16.51 (2 CH3), 16.10 (CH3), 16.00 (CH3).

Le même composé peut être obtenu avec un rendement de 10% en utilisant l'EDCI comme agent de couplage alors que la condensation entre le chlorure de l'acide squaloyle et le ddI le donne avec un rendement de 15%.

La taille des particules est évaluée selon le protocole décrit dans l'exemple 2. Le diamètre hydrodynamique moyen est de 152 nm, mesuré avec un écart type de 34,4nm et un indice de polydispersité de 0,1.

### Exemple 4 : Synthèse des nanoparticules de la 5'-squalenoyl-zidovudine; (2S,3S,5R)-4,8,13,17,21-Pentamethyl-docosa-4,8,12,16,20-pentaenoate de 3-azido-5-(5-méthyl-2,4-dioxo-3,4-dihydro-2H-pyrimidin-1-yl)-tétrahydro-furan-2-yl-méthyle

A une solution de 50 mg d'acide (4,8,13,17,21-pentaméthyl-docosa-4,8,12,16,20-pentaenoïque (SqCO2H, 0,15 mmol) dans le diméthylformamide anhydre (2 mL) sont ajoutés 45 mg de N-hydroxybenzotriazol ( 0,29 mmol), 79 mg de zidovudine (AZT, 0,24 mmol), 113 mg de O-(7-azabenzobiazol-1-yl)-N,N,N',N'-tetramethyluronium hexafluoroborate (0,29 mmol) et finalement 102 mg de diisopropyléthylamine (0,5 mmol). Le mélange est agité 90 h à 20 °C sous atmosphère d'azote, puis concentré sous pression réduite (0,05 Torr). Le résidu est repris dans 10 mL d'une solution aqueuse saturée en bicarbonate de sodium, et extrait à l'acétate d'éthyle (3 X 15 mL). La phase organique est lavée par une solution aqueuse de NaCl, séchée sur MgSO4, et concentrée sous vide. Le résidu est chromatographié sur gel de silice (CH2Cl2/MeOH: 97/3) pour fournir 52 mg de 5'-squalenoyl-zidovudine (Rdt 43%) sous la forme d'un solide amorphe incolore.

IR (cm-1) 3158, 2920, 2854, 2105, 1741, 1690, 1449, 1381, 1270.

¹H RMN (200 MHz, CDCl3) δ: 8.2 (s large, 1H), 7.22 (s, 1H), 6.12 (t, J = 6.4 Hz, 1H), 5,17-5,00 (m, 5H), 4,40 (dd, J = 12.2 Hz, 4.6 Hz, 1H), 4.30 (dd, 12.2 Hz, 3.8 Hz, 1H), 4.10-4.05 (m, 1H), 2,55-2.20 (m, 5 H), 2.10-1.90 (m, 18 H), 1.94 (s, 3H), 1,69 (s, 3H), 1.60 (s large, 15 H).

¹³C RMN (50 MHz, CDCl3) δ: 172.87 (CO2), 163.57 (CO), 150.12 (CO), 135.31 (C), 135.27 (CH), 135.04 (C), 134.91 (C), 132.86 (C), 131.35 (C), 125.79 (CH), 124.67 (CH), 124.56 (CH), 124.40 (CH), 124.37 (CH), 111.43 (C), 85.64 (CH), 82.00 (CH2), 63.36 (CH2), 60.81 (CH), 39.88 (CH2), 39.85 (CH2), 39.68 (CH2), 37.75 (CH2), 34.62 (CH2), 33.18 (CH2), 29.81 (CH2), 28.41 (CH2), 28.39 (CH2), 26.91 (CH2), 26.82 (CH2), 26.81 (CH2), 25.80 (CH3), 17.79 (CH3), 16.17 (2 CH3), 16.16 (CH3), 16.12 (CH3), 16.05 (CH3), 12.73 (CH3).

La taille des particules est évaluée selon le protocole décrit dans l'exemple 2. Le diamètre hydrodynamique moyen est de 150-170 nm.

### Exemple 58: Préparation des nanoparticules constituées de 4-(N)-squalénoylacyclovir (SQACV)

Le 4-(*N*)-squalénoylacyclovir est synthétique à partir de l'acide squalénique par réaction avec l'acyclovir. Selon la procédure utilisée, il est possible d'obtenir entre la chaîne squalénique et l'acyclovir soit une liaison ester, soit une amidique. Les particules contituées de SQACV sont obtenues selon la technique de la nanoprécipitation, comme décrit pour les particules de SQgem, et leur diamètre hydrodynamique moyen est de 217,5 + 37,9 nm pour une concentration en SQACV dans la suspension finale de. 1 mg/ml (index de polydispersité : 0,038).

## Revendications

1. Utilisation de l'acide squalénique ou d'un dérivé hydrocarboné au moins en C18, acyclique, non linéaire et insaturé du radical squalénoyle apte à se compacter lorsqu'il est mis en présence d'un solvant polaire pour formuler au moins un principe actif de nature polaire et de poids moléculaire supérieur à 100 Da à l'état de nanoparticules, ledit principe actif étant un nucléoside ou l'un de ses analogues.

2. Utilisation selon la revendication 1, **caractérisée en ce que** le poids moléculaire est supérieur à 150 Da.

3. Utilisation selon l'une des revendications 1 ou 2, **caractérisée en ce que** le poids moléculaire est supérieur à 200 Da.

4. Utilisation selon l'une quelconque des revendications précédentes, dans laquelle au moins une molécule dudit acide squalénique ou dudit dérivé est couplée de manière covalente à une molécule dudit principe actif.

5. Utilisation selon l'une quelconque des revendications précédentes, **caractérisée en ce que** ladite molécule de principe actif liée de manière covalente à au moins une molécule d'acide squalénique ou dudit dérivé est formulée à l'état de nanoparticules.

6. Utilisation selon la revendication précédente, dans laquelle lesdites nanoparticules possèdent une taille moyenne variant de 30 à 500 nm, en particulier de 50 à 250 nm, notamment de 100 à 175 nm.

7. Utilisation selon l'une quelconque des revendications précédentes, **caractérisée en ce que** ledit principe actif est un dérivé de la 2',2'-difluoro-2'-désoxycytidine.

8. Utilisation selon l'une quelconque des revendications 1 à 6, **caractérisée en ce que** ledit principe actif est choisi parmi la cytarabine, la didanosine, la zidovudine, l'acyclovir, la zalcitabine, le gancyclovir, le valacyclovir, la stavudine, la lamivudine, l'abacavir, l'emtricitabine, l'amdoxovir, le 2'-déoxy-3'-oxa-4'-thiocytidine (dOTC) et le sidophovir.

9. Utilisation selon la revendication 8, **caractérisée en ce que** ledit principe actif est la cytarabine.

10. Utilisation selon la revendication 8, **caractérisée en ce que** ledit principe actif est la didanosine.

11. Utilisation selon la revendication 8, **caractérisée en ce que** ledit principe actif est la zidovudine.

12. Utilisation selon la revendication 8, **caractérisée en ce que** ledit principe actif est l'acyclovir.

13. Nanoparticules formées d'au moins une molécule d'acide squalénique ou un dérivé hydrocarboné au moins en C18, acyclique, non linéaire et insaturé du radical squalénoyle apte à se compacter lorsqu'il est mis en présence d'un solvant polaire couplé de manière covalente à une molécule d'un principe actif de nature polaire et de poids moléculaire supérieur à 100 Da, ledit principe actif étant un nucléoside ou l'un de ses analogues.

14. Nanoparticules selon la revendication. 13 étant telles que définies en revendications 5 à 12.

15. Nanoparticules de 4-(N)-squalénoyl-cytazabine.

16. Nanoparticules de 5'-squalénoyl-didanosine.

17. Nanoparticules de 5'-squalénoyl-zidovudine.

18. Nanoparticules de 4-(N)-squalénoyl-acyclovir.

19. Composition pharmaceutique comprenant à titre de matière active au moins des nanoparticules selon l'une quelconque des revendications 13 à 18.

## Claims

1. Use of squalenic acid or of an unsaturated, nonlinear, acyclic, at least C18 hydrocarbon-based derivative of the squalenoyl radical capable of becoming compacted when it is placed in the presence of a polar solvent, for formulating at least one active ingredient which is of polar nature and which has a molecular weight of greater than 100 Da, in the nanoparticle state, said active ingredient being a nucleoside or an analogue thereof.

2. Use according to Claim 1, **characterized in that** the molecular weight is greater than 150 Da.

3. Use according to either of Claims 1 and 2, **characterized in that** the molecular weight is greater than 200 Da.

4. Use according to any one of the preceding claims, in which at least one molecule of said squalenic acid or of said derivative is covalently coupled to a molecule of said active ingredient.

5. Use according to any one of the preceding claims, **characterized in that** said molecule of active ingredient covalently bonded to at least one molecule of squalenic acid or of said derivative is formulated in the nanoparticle state.

6. Use according to the preceding claim, in which said nanoparticles have an average size ranging from 30 to 500 nm, in particular from 50 to 250 nm, especially from 100 to 175 nm.

7. Use according to any one of the preceding claims, **characterized in that** said active ingredient is a 2',2'-difluoro-2'-deoxycytidine derivative.

8. Use according to any one of Claims 1 to 6, **characterized in that** said active ingredient is chosen from cytarabine, didanasine, zidovudine, acyclovir, zalcitabine, gancyclovir, valacyclovir, stavudine, lamivudine, abacavir, emtricitabine, amdoxovir, 2'-deoxy-3'-oxa-4'-thiocytidine (dOTC) and sidophovir.

9. Use according to Claim 8, **characterized in that** said active ingredient is cytarabine.

10. Use according to Claim 8, **characterized in that** said active ingredient is didanosine.

11. Use according to Claim 8, **characterized in that** said active ingredient is zidovudine.

12. Use according to Claim 8, **characterized in that** said active ingredient is acyclovir.

13. Nanoparticles formed from at least one molecule of squalenic acid or an unsaturated, nonlinear, acyclic, at least C18 hydrocarbon-based derivative of the squalenoyl radial capable of becoming compacted when it is placed in the presence of a polar solvent, covalently coupled to a molecule of an active ingredient which is of polar nature and which has a molecular weight of greater than 100 Da, said active ingredient being a nucleoside or an analogue thereof.

14. Nanoparticles according to Claim 13, which are as defined in Claims 5 to 12.

15. Nanoparticles of 4-(N)-squalenoyl-cytarabine.

16. Nanoparticles of 5'-squalenoyl-didanosine.

17. Nanoparticles of 5'-squalenoyl-zidovudine.

18. Nanoparticles of 4-(N)-squalenoyl-acyclovir.

19. Pharmaceutical composition comprising, as active material, at least nanoparticles according to any one of Claims 13 to 18.

## Patentansprüche

1. Verwendung von Squalensäure oder eines acyclischen, nicht-linearen und ungesättigten Kohlenwasserstoffderivats mit mindestens C18 des Squalenoylrests, welches befähigt ist, in Gegenwart eines polaren Lösungsmittels kompakt zu werden, zur Formulierung mindestens eines Wirkstoffs polarer Natur und mit einem Molekulargewicht von größer als 100 Da im Zustand von Nanoteilchen, wobei der Wirkstoff ein Nucleosid oder ein Analogon davon ist.

2. Verwendung nach Anspruch 1, **dadurch gekennzeichnet, dass** das Molekulargewicht größer als 150 Da ist.

3. Verwendung nach einem der Ansprüche 1 oder 2, **dadurch gekennzeichnet, dass** das Molekulargewicht größer als 200 Da ist.

4. Verwendung nach einem der vorhergehenden Ansprüche, wobei mindestens ein Molekül der Squalensäure oder des Derivats kovalent an ein Molekül des Wirkstoffs gebunden ist.

5. Verwendung nach einem der vorhergehenden Ansprüche, **dadurch gekennzeichnet, dass** das Molekül des Wirkstoffs, welches kovalent an mindestens ein Molekül der Squalensäure oder des Derivats davon gebunden ist, im Zustand von Nanoteilchen formuliert ist.

6. Verwendung nach dem vorhergehenden Anspruch, wobei die Nanoteilchen eine mittlere Größe von 30 bis 500 nm aufweisen, insbesondere von 50 bis 250 nm, ganz besonders von 100 bis 175 nm.

7. Verwendung nach einem der vorhergehenden Ansprüche, **dadurch gekennzeichnet, dass** der Wirkstoff ein Derivat von 2',2'-Difluor-2'-desoxycytidin ist.

8. Verwendung nach einem der Ansprüche 1 bis 6, **dadurch gekennzeichnet, dass** der Wirkstoff aus Cytarabin, Didanosin, Zidovudin, Aciclovir, Zalcitabin, Ganciclovir, Valaciclovir, Stavudin, Lamivudin, Abacavir, Emtricitabin, Amdoxovir, 2'-Deoxy-3'-oxa-4'-thiocytidin (dOTC) und Sidophovir ausgewählt ist.

9. Verwendung nach Anspruch 8, **dadurch gekennzeichnet, dass** der Wirkstoff Cytarabin ist.

10. Verwendung nach Anspruch 8, **dadurch gekennzeichnet, dass** der Wirkstoff Didanosin ist.

11. Verwendung nach Anspruch 8, **dadurch gekennzeichnet, dass** der Wirkstoff Zidovudin ist.

12. Verwendung nach Anspruch 8, **dadurch gekennzeichnet, dass** der Wirkstoff Aciclovir ist.

13. Nanoteilchen, gebildet aus mindestens einem Squalensäuremolekül oder einem acyclischen, nicht-linearen und ungesättigten Kohlenwasserstoffderivat mit mindestens C18 des Squalenoylrests, welches befähigt ist, in Gegenwart eines polaren Lösungsmittels kompakt zu werden und kovalent an ein Molekül eines Wirkstoffs polarer Natur gebunden ist, und mit einem Molekulargewicht von größer als 100 Da, wobei der Wirkstoff ein Nucleosid oder ein Analogon davon ist.

14. Nanoteilchen nach Anspruch 13, wobei diese wie in den Ansprüchen 5 bis 12 definiert sind.

15. Nanoteilchen von 4-(N)-Squalenoyl-cytarabin.

16. Nanoteilchen von 5'-Squalenoyl-didanosin,

17. Nanoteilchen von 5'-Squalenoyl-zidovudin.

18. Nanoteilchen von 4-(N)-Squalenoyl-aciclovir.

19. Pharmazeutische Zusammensetzung, welche als aktive Substanz zumindest die Nanoteilchen nach einem der Ansprüche 13 bis 18 enthält.
